Europäisches Patentamt

European Patent Office

Office européen des brevets

(11) Veröffentlichungsnummer : **0 411 274 B1**

(12) **EUROPÄISCHE PATENTSCHRIFT**

(45) Veröffentlichungstag der Patentschrift :
**25.11.92 Patentblatt 92/48**

(21) Anmeldenummer : **90110635.1**

(22) Anmeldetag : **05.06.90**

(51) Int. Cl.⁵ : **B01L 3/14,** G01N 33/04

㊴ **Flasche.**

(30) Priorität : **28.07.89 DE 3925165**

(43) Veröffentlichungstag der Anmeldung :
**06.02.91 Patentblatt 91/06**

(45) Bekanntmachung des Hinweises auf die
Patenterteilung :
**25.11.92 Patentblatt 92/48**

(84) Benannte Vertragsstaaten :
**AT BE CH DE DK ES FR GB GR IT LI LU NL SE**

(56) Entgegenhaltungen :
**EP-A- 0 030 562**
**WO-A-87/00509**
**DE-A- 2 758 437**
**FR-A- 2 357 437**
**US-A- 3 927 782**

(73) Patentinhaber : **ULTRAKUST ELECTRONIC
GmbH
Sudetenstrasse 5-7
W-8375 Ruhmannsfelden (DE)**

(72) Erfinder : **Glasschröder, Johann
Jahnstrasse 4
W-8375 Ruhmannsfelden (DE)**
Erfinder : **Lehmann, Lutz
Hofgraben 470
W-8910 Landsberg/Lech (DE)**
Erfinder : **Völkl, Franz
Lackerbauerstrasse 14
W-8374 Viechtach (DE)**

(74) Vertreter : **Heim, Hans-Karl, Dipl.-Ing. et al
c/o Weber & Heim Hofbrunnstrasse 36
W-8000 München 71 (DE)**

## Beschreibung

Die Erfindung betrifft eine Flasche zur vorübergehenden Aufnahme eines zu prüfenden Mediums, insbesondere einer Milchprobe eines Lieferanten gemäß Oberbegriff des Anspruchs 1.

Eine Flasche der gattungsgemäßen Art ist aus der DE-PS 27 58 437 bekannt. Diese Flasche hat zur Identifikation einen an ihrem Umfang angeordneten Magnetspeicher und an ihrem Boden eine ferromagnetische Platte mit einer Markierung. Eine derartige Flasche hat sich im praktischen Einsatz bewährt, wobei jedoch für die Befestigung der ferromagnetischen Platte an der Flasche ein verfahrenstechnisch aufwendiger Klebevorgang notwendig ist.

Des weiteren sind aus der US-PS 3,927,782, der FR-23 57 437 A1 oder der WO 87/00509 Flaschen bekannt, die zur Aufnahme von Flüssigkeiten, welche unter Druck stehen oder unter Druck eingefüllt werden, geeignet sind. Bei diesen Flaschen handelt es sich im wesentlichen um relativ dünnwandige Flaschen aus einem Kunststoff, die ihre Stabilität erst durch die eingefüllte Flüssigkeit erhalten. Diese am unteren Bereich abgerundet ausgebildeten Flaschen weisen an ihrem unteren Bereich speziell zur Verbesserung der Standfähigkeit auf einer ebenen Fläche ein topfförmiges Element auf, das mit einem periphär umlaufenden Befestigungsbereich oder mit einem am Boden der Flasche vorgesehenen Zapfen zur Halterung mit dem topfförmigen Element ausgestattet ist.

Diese bekannten Ausführungsformen der Flaschen haben jedoch den Nachteil, daß einerseits am oberen Bereich des topfförmigen Elements keine Dichtung vorhanden ist, die ein Eindringen von Flüssigkeit, Schmutz oder dergleichen am oberen Bereich verhindert. Andererseits sind auch im leeren oder weitgehend leeren Zustand dieser Flaschen Stabilitätsprobleme durch den nicht vorhandenen inneren Druck in der Flasche möglich.

Als unzureichend müssen jedoch die Zentrierungsaspekte der Flasche gegenüber dem topfförmigen Element angesehen werden, insbesondere wenn es sich um kleine Flaschen handelt, die auf automatischen Förderanlagen transportiert und z.B. Prüftests auf ihren Inhalt an Laborstationen durchlaufen sollen. In dieser Hinsicht und im Hinblick auf relativ hohe Temperaturunterschiede weisen die bekannten Flaschen bei den Abdichtungsaspekten und ihrem festen Sitz beträchtliche Nachteile auf.

Der Erfindung liegt daher die Aufgabe zugrunde, eine Flasche der gattungsgemäßen Art so zu konzipieren, daß in kostengünstiger, einfacher Weise eine einwandfreie Verbindung zwischen der Flasche und einem Stell- bzw. Beförderungsmittel erreicht wird, wobei hygienische und Zentrierungsaspekte Berücksichtigung finden sollen.

Diese Aufgabe wird erfindungsgemäß bei einer Flasche der gattungsgemäßen Art durch die Merkmale des kennzeichnenden Teils des Anspruchs 1 gelöst.

Ein wesentlicher Gedanke der Erfindung kann darin gesehen werden, den unteren Bereich der Flasche zunächst durchmesserreduziert auszubilden und in diesem Bereich eine primär Haltefunktion übernehmende Halteleiste, eine Dichtungszwekken dienende Dichtleiste und eine radial verlaufende Anschlagfläche für die obere Kante des topfbzw. ringförmigen Elements vorzusehen. Durch diese konstruktiven Merkmale wird einerseits eine zentrierende Ausrichtung beim Einpressen der Flasche in das topfbzw. ringförmige Element erreicht, wobei nach dem Einsetzvorgang der Flasche in das Element die überwiegende Haltefunktion von der unteren Halteleiste erzeugt wird, die in Art einer Spreizung einer Tellerfeder die Haltekräfte auf die Innenwand des topfförmigen Elements aufbringt.

Dieses Zusammenwirken der einzelnen Elemente stellt sich in besonders vorteilhafter weise bei einer Kunststofflasche und einem aus magnetisierbaren Material bestehenden topf- bzw. ringförmigen Element heraus.

Die Mittel und die Flasche können in einem Preß- und/oder Drehvorgang miteinander verbunden werden. Es läßt sich somit eine schnelle und kostengünstige Verbindung der beiden Teile erreichen. Auf diese Weise können kostengünstige Kunststofflaschen als Einwegflaschen verwendet werden, wobei die Mittel nach Gebrauch wieder von der Flasche gelöst werden. Es ist jedoch auch möglich, die Mittel fest und unlösbar mit der Flasche zu verbinden, insbesondere wenn diese als Mehrwegflaschen ausgebildet sind.

Die Mittel sind geeigneterweise an einem topfförmigen Element ausgebildet, dessen Topfwand den Umfangsbereich am unteren Ende der Flasche umgreift und dessen Topfboden als Standfläche für die Flasche ausgebildet ist. Ein derartiges Element kann auf einfache Weise auf die Flasche aufgepreßt oder aufgedreht werden. Im letzteren Fall weist der Umfangsbereich der Flasche ein Gewinde auf, welches mit einem an der Innenseite der Topfwand ausgebildeten Innengewinde in Eingriff bringbar ist.

In einer weiteren Ausführungsform hat der Umfangsbereich der Flasche mindestens eine in Umfangsrichtung verlaufende ringförmige Rippe oder Leiste, in die mindestens ein auf der Innenseite der Topfwand ausgebildeter Vorsprung eingreift. Bei dieser Ausführungsform lassen sich die Flasche und das topfförmige Element sehr gut miteinander verpressen, wobei eine sehr feste Verbindung zwischen der Flasche und dem topfförmigen Element erreicht wird, wenn die Rippen oder Leisten an ihrer oberen Seite eine im wesentlichen radial verlaufende Rastfläche aufweisen. Wenn die Flasche und das topfförmige Element zusammengedrückt werden, greift somit der Vorsprung an der Innenseite der Topfwand hinter diese radial verlaufende Rastfläche und bewirkt so einen festen und dichten Sitz des topfförmigen Elements an der Flasche.

Um einen festen Sitz der Flasche auf einer Transporteinrichtung zu gewährleisten, ist es vorteilhaft, wenn das topfförmige Element zumindest am Topfboden eine ferromagnetische Platte aufweist. Diese wirkt mit an der Transportvorrichtung ausgebildeten Magneten zusammen und ermöglicht einen zuverlässigen Transport auch von einzelnen Flaschen.

In einer weiteren Ausbildungsform steht vom Boden der Flasche ein axialer, zentraler, pilzförmiger Stift ab, der mit einem Randbereich um eine im Topfboden angeordnete zentrische Öffnung in Eingriff bringbar ist. Der pilzförmige Stift der Flasche hat einen erweiterten Kappenbereich, dessen Durchmesser größer ist als der der zentrischen Öffnung. Beim Zusammendrücken von topfförmigem Element und Flasche wird der erweiterte Kappenbereich des pilzförmigen Stifts durch die Öffnung hindurchgedrückt und hintergreift dann den Randbereich des topfförmigen Elements um die zentrische Öffnung. Wenn der Randbereich in axialer Richtung zur Flasche hin versetzt ist, behindert der pilzförmige Stift nicht die Standeigenschaften der Flasche.

Eine weitere Verbindungsmöglichkeit ist dadurch gegeben, daß der Umfangsbereich der Flasche um deren Achse schraubenförmig geneigte Rampenflächen aufweist, die mit auf der Innenseite der Topfwände ausgebildeten Vorsprüngen in Eingriff bringbar sind. Das topfförmige Element ist hierdurch in der Art eines Marmeladenglasdeckels mit der Flasche verschraubbar.

Selbstverständlich können die Mittel auch an einem ringförmigen Element ausgebildet sein. Das ringförmige Element ist hierbei als ein topfförmiges Element ohne Topfboden anzusehen, wobei die oben genannten mit dem Umfangsbereich der Flasche zusammenwirkenden Verbindungseinrichtungen des topfförmigen Elements auch bei dem ringförmigen Element realisiert werden können.

Bei der Flasche gemäß der Erfindung ist der Umfangsbereich am unteren Ende der Flasche relativ zum übrigen Umfangsbereich durchmesserverringert und von dem topf- oder ringförmigen Element überdeckbar. Der Außendurchmesser des topf- bzw. ringförmigen Elements entspricht hierbei dem Außendurchmesser des weiter oben gelegenen Flaschenbereichs. Auf diese Weise entstehen an der Umfangsfläche der Flasche keine Vorsprünge, an denen sich Schmutz ansammeln könnte. Gerade beim Umgang mit verderblichen Milchprodukten sind im Hinblick auf hohe Hygieneanforderungen glatte und strukturlose Formen vorteilhaft.

In einer vorteilhaften Weiterbildung der Erfindung hat die Flasche in ihrem unteren Umfangsbereich mindestens eine obere Leiste und mindestens eine untere Leiste, wobei die obere Leiste an der Innenseite der Topfwand dicht anliegt und wobei der sickenartig an der Topfwand ausgebildete Vorsprung über der unteren Leistung einrastbar ist.

Das topfförmige Element hat hierfür vorzugsweise vier sikkenförmig ausgebildetete Vorsprünge, die an dem Topfrand um 90° versetzt angeordnet sind. Beim Aufpressen des topfförmigen Elements auf die Flasche, z.B. von Hand, gleiten die vier Vorsprünge über die untere Leiste und verrasten an der nach oben weisenden radial verlaufenden Rastfläche der unteren Leiste. Diese ist vorzugsweise an ihrer Außenseite abgerundet, um ein besseres Übergleiten der Vorsprünge zu ermöglichen und um den Anpreßdruck der Vorsprünge auf die Rastfläche der unteren Leiste großflächiger zu verteilen. Die obere Leiste ist vorzugsweise zu ihrer Außenseite hin zugespitzt und liegt an der Innenseite der Topfwand dicht an, so daß keine Flüssigkeit durch die Kontaktstelle der Topfwand mit der oberen Leiste zwischen die Flasche und das topfförmige Element gelangen kann. Durch das Vorsehen von zwei oberen und zwei unteren Leisten ist das topfförmige Element relativ zur Flasche in zwei axial unterschiedliche Stellungen einrastbar.

Wenn zwischen dem oberen Umfangsbereich der Flasche und dem diesen gegenüber durchmesserverringerten unteren Umfangsbereich eine radial verlaufende, nach unten weisende Dichtfläche ausgebildet ist, an der die obere Kante des topf- und ringförmigen Elements dicht zu liegen kommt, wird zusätzlich ein Eindringen von Milch zwischen die Flasche und das topf- bzw. ringförmige Element verhindert, was im Sinne hoher Hygieneanforderungen wünschenswert ist. Das zwischen Dichtfläche und unterer Leiste befindliche Teil der Topfwand ist elastisch zwische diesen eingespannt, wobei durch die Abrundung der unteren Leiste die Anpreßkraft der Sicken gleichmäßiger verteilt wird. Auf diese Weise wird eine elastische und damit langzeitbeständige Dichtfunktion gewährleistet.

Vorteilhafte Weiterbildungen der Erfindung sind Gegenstand der Unteransprüche.

Die Erfindung wird nachfolgend beispielsweise anhand der schematischen Zeichnung beschrieben. In dieser zeigen:

Fig. 1 eine teilgeschnittene Seitenansicht einer Milchprobenflasche mit einem topfförmigen Element für den Transport der Flasche;

Fig. 2 eine Ansicht gemäß Fig. 1 ohne das topfförmige Element;

Fig. 3 einen vergrößerten Ausschnitt III aus Fig. 1;

Fig. 4 einen vergrößerten Ausschnitt IV aus Fig. 2;

Fig. 5 eine geschnittene Seitenansicht des Bodenbereichs mit einem topfenförmigen Element und einer magnetisierbaren Platte, wobei in Fig. 5 und den weiteren Figuren im wesentlichen auf Beispiele zur Befestigung von Flasche und Element abgestellt ist;

Fig. 6 eine geschnittene Seitenansicht des Bodenbereichs der Flasche mit einem ringförmigen Element;

Fig. 7 eine geschnittene Seitenansicht gemäß Fig. 6; und

Fig. 8 eine geschnittene Seitenansicht des Bodenbereichs der Flasche mit einem topfförmigen Element, das von einem an der Flasche ausgebildeten Fortsatz hintergriffen wird.

Fig. 9 eine teilgeschnittene Seitenansicht des Bodenbereichs einer Flasche mit schraubenförmig geneigten Rampenflächen;

Fig. 10 eine geschnittene Seitenansicht des Bodenbereichs einer Flasche mit konvex gekrümmter Bodenfläche;

Fig. 11 eine geschnittene Seitenansicht eines topfförmigen Elementes für den Transport einer Flasche und

Fig. 12 einen Schnitt längs der Linie XII-XII aus Fig. 11.

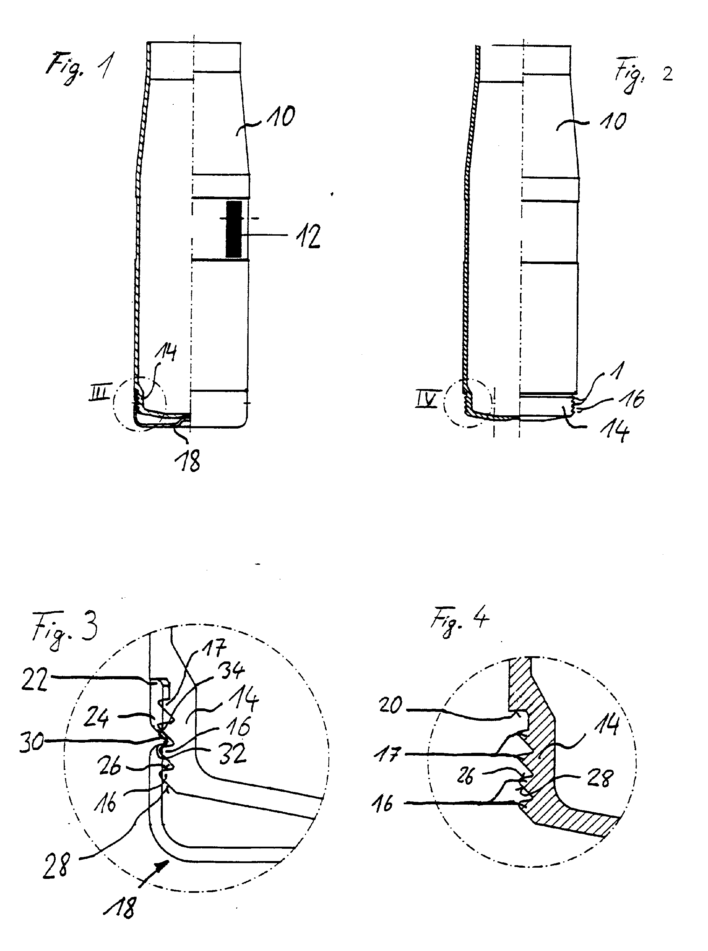

In Fig. 1 und 2 ist eine kreiszylindrische Milchprobenflasche 10 dargestellt, auf deren mittlerem Umfangsbereich ein oder mehrere Bar-Codes 12 zur Identifizierung der Milchprobe angeordnet sind. Die Anordnung zweier Bar-Codes 12 verringert beträchtlich den Ausfall aufgrund schlechter Lesbarkeit eines Einzel-Bar-Codes. Im Bodenbereich ist die Flasche relativ zum übrigen Bereich durchmesservermindert und trägt mehrere in Umfangsrichtung verlaufende ringförmige Rippen 16,17. Auf den durchmesserverminderten Umfangsbereich 14 wird ein topfförmiges Element 18 zum Transport der Flasche aufgepreßt. Das topfförmige Element ist nachfolgend noch in näher beschriebener Weise mit der Milchflasche 10 verbindbar.

Fig. 3 und 4 zeigen vergrößerte Ausschnitte des Bodenbereichs der Milchprobenflasche (Fig. 4) und der Verbindungsstelle der Milchprobenflasche mit dem topfförmigen Element (Fig. 3).

Fig. 4 zeigt den durchmesserverminderten unteren Umfangsbereich 14 der Flasche 10, der an seiner äußeren Umfangsfläche axial versetzt zwei untere Leisten 16 und zwei obere Leisten 17 aufweist. Die oberen Leisten 17 sind zu ihrer Außenseite hin zugespitzt und für die Anlage an dem topfförmigen Element 18 ausgebildet. Die unteren Leisten 16 sind an ihrer Außenseite abgerundet und als Rastelemente für das topfförmige Element 18 ausgebildet. Zwischen dem durchmesserverminderten Umfangsbereich 14 und dem restlichen Umfangsbereich der Flasche ist eine radial verlaufende nach unten gerichtete Anlage- und Dichtfläche 20 ausgebildet.

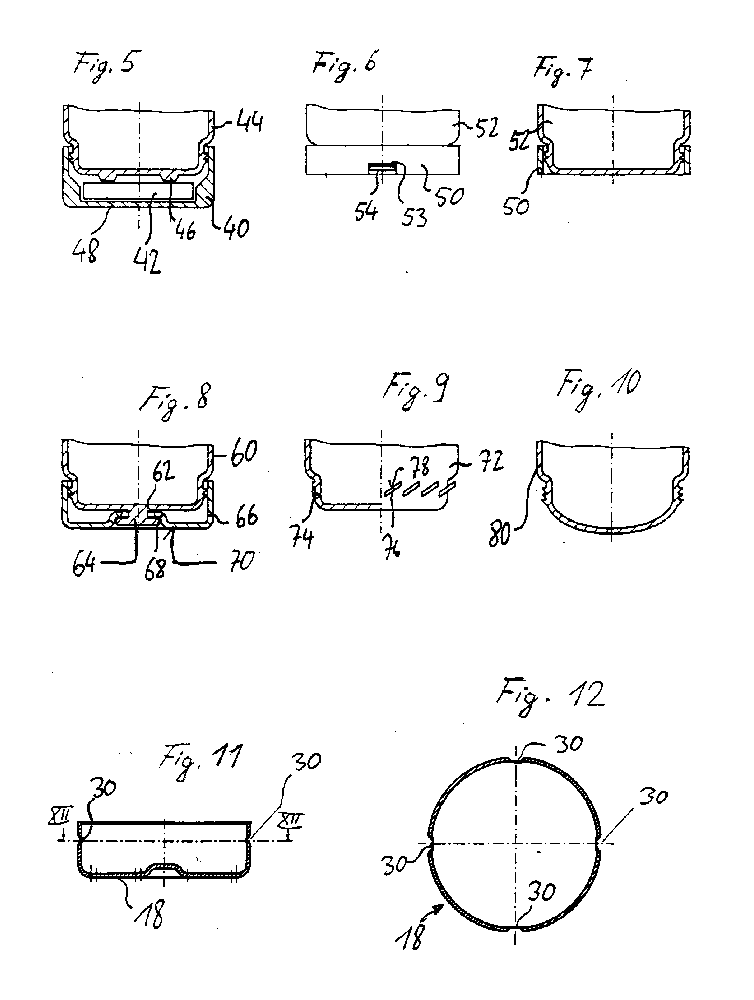

Beim Verpressen (maschinell oder von Hand) kommt die Topfwand 24 des topfförmigen Elements 18 über dem durchmesserverminderten Umfangsbereich 14 zu liegen (Fig. 3). Auf der Innenseite der Topfwand sind vier um 90° versetzte (Fig. 11 und 12) als sickenförmige Vorsprünge ausgebildete Rastnasen 30 angeordnet. Diese Rastnasen 30 gleiten beim Verpressen von topfförmigem Element 18 und Flasche 10 über die Außenseiten der unteren Leisten 16, bis die obere Kante 22 der Topfwand 24 an der Anlage- oder Dichtfläche 20 zu liegen kommt. Die Rastnase 30 ist an ihrer dem Topfboden zugewandten Seite 32 als im wesentlichen radiale Fläche ausgebildet, wohingegen die der Flasche 10 zugewandte Seite 34 der Rastnase 30 als zum Boden des topfförmigen Elements 18 hin verjüngte konische Fläche 34 ausgebildet ist. Die unteren Leisten 16 haben ebenfalls eine nach oben weisende als Rastfläche radial verlaufende obere Seite 26 , die über einen abgerundeten Außenbereich in eine auf die Mitte des Flaschenbodens konisch zulaufende, als Gleitfläche ausgebildete untere Seite 28 übergeht. Durch die konische Ausbildung der Oberseite 34 der Rastnasen 30 und der Unterseiten 28 der unteren Leisten 16 wird ein leichtes Zusammenpressen von Flasche und topfförmigem Element erreicht. Beim Aufeinanderpressen können die unteren Leisten 16 durch die Rastnasen 30 verletzt werden, so daß die Haltekräfte zwischen der Flasche 10 und dem topfförmigen Element 18 geschmälert werden. Dies wird verhindert, indem nach dem Verpressen von Flasche 10 und topfförmigem Element 18 diese leicht zueinander verdreht werden. Die Verletzung der unteren Leisten 16 hat den positiven Effekt, daß die in dem Topf beim Verpressen komprimierte Luft zumindest teilweise entweichen kann, bis die nächst höher gelegene Leiste 16,17 den Topf verschließt. Die durch den Druck der Luftkompression erzeugte axiale Gegenkraft wird so abgebaut.

Im verpreßten Zustand ist die radiale Fläche 32 der Rastnase 30 gegen die als Rastfläche ausgebildete radiale Oberseite 26 der unteren Leiste 16 abgestützt. Der obere Bereich der Topfwand 24 ist somit zwischen der Anlage- und Dichtfläche 20 und den Rastnasen 30 elastisch eingespannt. Ein Dichteffekt wird zum einen dadurch erreicht, daß die spitzen Außenseiten der oberen Leisten 17 an der Innenseite der Topfwand 24 anliegen und gegebenenfalls sogar ein wenig in diese einschneiden. Ein weiterer Dichteffekt wird durch die Anlage der oberen Kante 22 der Topfwand 24 an der Anlage- und Dichtfläche 20 erzielt.

Das Übermaß der Leisten 16,17 gegenüber dem Topfdurchmesser wird solange angepaßt, bis der erwünschte Anpreßdruck erreicht ist. Dieser wird so gewählt, daß die verpreßten Leisten 16, 17 gerade noch federelastisch bleiben und sich noch nicht dauerhaft verformen (Kaltfluß). In Fig. 3 werden die übermäßigen Leisten 16,17 entgegen der Wirklichkeit unverformt dargestellt.

Das topfförmige Element 18 ist in den Fig. 11 und 12 dargestellt. Wie in dem Schnitt in Fig. 12 zu sehen ist, sind an dem topfförmigen Element 18 vier sickenförmige Rastnasen 30 ausgebildet.

Die Fig. 5 bis 10 zeigen alternative Ausführungsformen des Bodenbereichs der Milchflasche bzw. der Mittel zum Ausrichten und/oder Transportieren der Flasche. In Fig. 5 hat das topfförmige Element 40 eine ferromagnetische Platte 42, die im auf eine Flasche 44 aufgepreßten Zustand zwischen an dem Boden der Flasche 44 ausgebildeten Fortsätzen 46 und dem Topfboden 48 des topfförmigen Elements 40 zu liegen kommt. Auf diese Weise wird eine feste Verbindung des topfförmigen Elements 40 und somit der Flasche 44 zu einer nicht dargestellten magnetischen Transporteinrichtung geschaffen. In diesem Fall wird die Platte lose in den Topf gelegt, nicht von ihm umspritzt. Das Herstellverfahren ist zeit- und kostensparend, es erlaubt zudem die Verwendung nicht gegen Rost geschützter Werkstoffe für die Scheibe und vermeidet ferner die Gefahr des Abplatzens der Scheibe vom (harten) Kunststofftopf, verursacht durch Temperatursprünge (-25° C im Winter im Freien, +120° C bei der Reinigung im Prüflabor).

Wie Fig. 6 und 7 zeigen, können die Mittel zum Ausrichten bzw. Transport einer Flasche auch als ringförmiges Element 50 ausgebildet sein, wobei die Verbindung zwischen dem ringförmigene Element 50 und der Flasche 52 im wesentlichen gemäß Fig. 3 und 4 erfolgen würde. Das ringförmige Element 50 hat an seiner Unterkante zwei um 180° versetzte Ausnehmungen 53, die von an der Flasche 52 ausgebildeten Schnappnasen 54 hintergriffen werden. In Fig. 8 ist eine zu dem bisher gezeigten Verbindungsmechanismus additive oder alternative Ausführungsform dargestellt. Eine Flasche 60 hat im Zentrum ihres Bodens 62 einen als pilzförmige Ausstülpung ausgebildeten Stift 64, der ein topfförmiges Element 66 an dessen um eine zentrale Öffnung ausgebildeten Randbereich 68 formschlüssig hintergreift. Der Randbereich 68 des topfförmigen Elementes 66 ist dabei in axialer Richtung der Flasche zu dieser hin versetzt, wodurch eine plane Standfläche 70 erhalten bleibt. Die Topfwände des topfförmigen Elementes 66 können zusätzlich in der in den Fig. 3 und 4 beschriebenen Weise mit der Flasche 60 verbunden sein.

Die Flasche 72 aus Fig. 9 weist in ihrem Bodenbereich eine durchmesserverminderte Umfangsfläche 74 auf, an der Leisten 76 mit schrägen Rampenflächen 78 angeordnet sind, die gleichmäßig um die Umfangsfläche verteilt schraubenförmig um die Flaschenachse angeordnet sind. Diese Rampenflächen 78 wirken mit an dem topfförmigen Element ausgebildeten Rastnasen 30 in der Art eines Marmeladenglasverschlusses zusammen, so daß das topf- oder ringförmige Element 18,40,50 durch eine Drehung relativ zur Flasche 72 fest mit dieser verbunden wird. Diese Verbindung ist wieder lösbar, so daß die Flasche 72 und das Element getrennt gereinigt werden können.

In Fig. 10 ist der Bodenbereich einer Milchflasche 80 dargestellt, die im wesentlichen der Milchflasche aus den Fig. 1 bis 4 entspricht, mit dem Unterschied, daß bei dieser Flasche 80 der Boden konvex nach unten gekrümmt ist. Hierdurch wird das zwischen dem topfförmigen Element 18 und der Flasche 80 vorhandene Luftvolumen auf ein Minimum reduziert, wodurch der beim Zusammenpressen der beiden Teile entstehende Gegendruck durch das zwischen Flasche 80 und topfförmige Element 18 vorhandene Luftpolster verringert wird.

Das in den Fig. 11 und 12 dargestellte topfförmige Element 18 mit den Rastelementen bzw. Rastnasen 30 kann im wesentlichen für die Flaschen 52,60,72,80 und 10 verwendet werden. Dies gilt in gleicher Weise für das ringförmige Element 50. In allen Ausführungsformen ist die Flasche vorzugsweise aus Kunststoff hergestellt und das ring- bzw. topfförmige Element besteht aus einem nicht-rostenden magnetisierbaren Metall, das sich tiefziehen läßt.

Die ringförmigen Rippen 16 aus den Fig. 3 und 4 können auch als Wulste ausgebildet sein. Durch das Vorsehen eines Bar-Codes anstelle eines Magnetstreifens kann die Flasche kürzer gehalten werden, wobei der gewonnene Raum genutzt werden kann, um den Flaschenboden gemäß Fig. 10 reagenzglasförmig abzurunden. In den Wänden bzw. am Boden des ring- bzw. topfförmigen Elementes können Stahleinlagen oder Magnete eingelagert sein, um die Verbindung des Elements mit einer Transporteinrichtung zu verbessern. In diesem Sinne kann die in Fig. 5 dargestellte Magnetplatte 42 auch als Stahlplatte ausgebildet sein.

Durch das topf- oder ringförmige Element kann die Flasche auf einem mit Einzelmagneten bestückten Transportband gehalten werden, z.B. wenn die Flaschen nach einer Analyse auf ein solches Band geschoben werden, um entleert und hängend in ein Reinigungsbad geführt zu werden, in dem sie von unten mit Reinigungsflüssigkeit ausgespritzt werden. In der Flasche kann daher in einem vollautomatisierten Betrieb eine Probe aufbewahrt werden, die beim Füllen, Analysieren und Reinigen in jeder gewünschten Lage betriebssicher gehalten werden kann.

Um ein leichtes Verpressen des topfförmigen Elementes mit der Flasche zu gewährleisten, sollte das Verhältnis des zwischen diesen eingeschlossenen Luftvolumens vor und nach dem Verpressen möglichst nahe bei 1 liegen.

**Patentansprüche**

1. Flasche zur vorübergehenden Aufnahme eines zu prüfenden Mediums, insbesondere einer Milchprobe eines Lieferanten, mit im Bodenbereich angeordneten Mitteln (18,50) für eine automatische Ausrichtung und den Transport der Flasche (10),
dadurch **gekennzeichnet,**

a) daß die Flasche (10) und die Mittel (18) einander formschlüssig hintergreifen,

b) daß die Mittel (18,50) als topf- oder ringförmiges Element (18) ausgebildet sind, das einen Umfangsbereich (14) am unteren Ende der Flasche (10) umgreift,

c) daß am unteren Umfangsbereich (14) der Flasche (10) mindestens eine peripher umlaufende obere Dichtleiste (17) und eine untere Halteleiste (16) zum Halten des Elements (18) an der Flasche (10) vorgesehen sind,

d) daß die Dichtleiste (17) relativ zum Innendurchmesser des Elements (18,50) ein Übermaß aufweist und federelastisch gegen dieses anliegt,

e) und daß die Flasche (10) am unteren Umfangsbereich (14) durchmesserverringert und mit einer radial verlaufenden Anlagefläche (20) ausgebildet ist, mit der die obere Kante (22) des Elements (18) in Dichtwirkung gelangt.

2. Flasche nach Anspruch 1,
dadurch **gekennzeichnet**,
daß die Mittel (18) aus einem magnetisierbaren, insbesondere tiefziehfähigen Material bestehen und die Flasche (10) aus einem Kunststoff.

3. Flasche nach Anspruch 1 oder 2,
dadurch **gekennzeichnet**,
daß die obere Dichtleiste (17) zu ihrer Außenseite hin zugespitzt und die untere Leiste (16) an ihrer Außenseite abgerundet ist.

4. Flasche nach einem der Ansprüche 1 bis 3,
dadurch **gekennzeichnet**,
daß zwei obere und zwei untere Leisten (17,16) vorgesehen sind.

5. Flasche nach einem der vorhergehenden Ansprüche,
dadurch **gekennzeichnet**,
daß der Topfboden des topfförmigen Elemtens (18) als Standfläche für die Flasche (10) ausgebildet ist.

6. Flasche nach einem der vorhergehenden Ansprüche,
dadurch **gekennzeichnet**,
daß das topfförmige Element (40) zumindest an seinem Topfboden (48) eine ferromagnetische Platte (42) aufweist.

7. Flasche nach einem der vorhergehenden Ansprüche,
dadurch **gekennzeichnet**,
daß der Außendurchmesser des topfförmigen Elements (18,50) dem Außendurchmesser des weiter oben gelegenen Umfangsbereichs der Flasche (10) entspricht.

8. Flasche nach einem der Ansprüche 1 bis 7,
dadurch **gekennzeichnet**,
daß die Leiste (16) an ihrer oberen Seite (26) eine im wesentlichen radial verlaufende Rastfläche aufweist, die mit einer an dem Vorsprung (30) ausgebildeten radial verlaufenden Rastfläche (32) in Eingriff tritt.

9. Flasche nach einem der vorhergehenden Asnprüche,
dadurch **gekennzeichnet**,
daß freie Ecken und Kanten der Flasche insbesondere in deren Innenraum und des topf- oder ringförmigen Elements abgerundet sind bzw. schräg nach innen abfallen.

10. Flasche nach einem der vorhergehenden Ansprüche,
dadurch **gekennzeichnet**,
daß mindestens ein auf der Innenseite der Topfwand (24) ausgebildeter sickenartiger Vorsprung (30) vorgesehen ist, der über der unteren Leiste (16) einrastet.

11. Flasche nach einem der vorhergehenden Ansprüche,
dadurch **gekennzeichnet**,
daß an der Innenseite der Topfwand (24) vier sicken- artige Vorsprünge (30) angeordnet sind.

## Claims

1. Bottle for the temporary reception of a medium to be tested, particularly a milk sample from a supplier, with means (18, 50) located in the bottom area for an automatic orientation and for the transportation of the bottle (10), characterized in that

a) the bottle (10) and the means (18) interlockingly engage behind one another,

b) the means (18, 50) are constructed as a cup-shaped or annular element (18), which engages round a circumferential area (14) at the lower end of the bottle (10),

c) on the lower circumferential area (14) of the bottle (10) is provided at least one peripherally passing round, upper sealing strip (17) and a lower retaining strip (16) for retaining the element (18) on the bottle,

d) the sealing strip (17) has an oversize relative to the internal diameter of the element (18, 50) and engages spring-elastically against the same,

e) and the bottle (10) has a reduced diameter on the lower circumferential area (14) and is constructed with a radially directed contact surface (20), with which the upper edge (22) of the element (18) comes into a sealing action.

2. Bottle according to claim 1, characterized in that the means (18) comprise a magnetizable, particularly a deep-drawable material and the bottle (10) is made from plastic.

3. Bottle according to claim 1 or 2, characterized in that the upper sealing strip (17) is tapered towards its outside and the lower strip (16) is rounded on its outside.

4. Bottle according to one of the claims 1 to 3, characterized in that two upper and two lower strips (17, 16) are provided.

5. Bottle according to one of the preceding claims, characterized in that the cup bottom of the cup-shaped element (18) is constructed as a standing surface for the bottle (10).

6. Bottle according to one of the preceding claims, characterized in that the cup-shaped element (40) has a ferromagnetic plate (42), at least on its cup bottom (48).

7. Bottle according to one of the preceding claims, characterized in that the external diameter of the cup-shaped element (18, 50) corresponds to the external diameter of the further upwardly positioned circumferential area of the bottle (10).

8. Bottle according to one of the claims 1 to 7, characterized in that the strip (16) has on its upper side (26) a substantially radially directed locking surface, which engages with a radially directed locking surface (32) formed on the projection (30).

9. Bottle according to one of the preceding claims, characterized in that free corners and edges of the bottle, particularly in its interior and of the cup-shaped or annular element are rounded or slope inwards.

10. Bottle according to one of the preceding claims, characterized in that at least one corrugation-like projection (30) is formed on the inside of the cup wall (24) and locks over the lower strip (16).

11. Bottle according to one of the preceding claims, characterized in that there are four corrugation-like projections (30) on the inside of the cup wall (24).

**Revendications**

1. Bouteille destinée à recevoir provisoirement un milieu à tester, en particulier un échantillon de lait d'un fournisseur, avec des moyens (18, 50)) placés au niveau de son fond pour l'orientation automatique et le transport de la bouteille (10), caractérisée en ce que,

   a) la bouteille (10) et les moyens (18) sont accrochés l'un à l'autre par interpénétration par la forme,

   b) les moyens (18, 50) sont réalisés en élément (18) en forme de pot ou d'anneau, qui enveloppe une zone périphérique (14) à la partie inférieure (10) de la bouteille,

   c) une nervure d'étanchéité supérieure (17) et une nervure de maintien inférieure (16) circulaires en périphérie et destinées à fixer l'élément (18) sur la bouteille (10) sont prévues dans la zone périphérique inférieure (14) de la bouteille (10),

   d) la nervure d'étanchéité (17) a une dimension plus grande que le diamètre intérieur de l'élément (18, 50) et appuie contre celui-ci en le déformant élastiquement,

   e) la bouteille (10) a un diamètre réduit à sa partie périphérique inférieure (14) et possède une surface d'appui (20) se développant radialement, avec laquelle le bord supérieur (22) de l'élément (18) crée un effet d'étanchéité.

2. Bouteille selon la revendication 1, caractérisée en ce que :
   - les moyens (18) sont constitués d'un matériau magnétisable, en particulier, apte à être embouti et la bouteille (10) est en matière synthétique.

3. Bouteille selon la revendication 1 ou la revendication 2, caractérisée en ce que :
   - la nervure supérieure d'étanchéité (17) est taillée en pointe à son extrémité extérieure et la nervure inférieure (16) est arrondie sur sa face extérieure.

4. Bouteille selon l'une des revendications 1 à 3, caractérisée en ce que :
   - on a prévu deux nervures supérieures et deux nervures inférieures (17, 18).

5. Bouteille selon l'une des revendications précédentes, caractérisée en ce que :
   - le fond de l'élément (18) en forme de pot sert de surface d'appui pour la bouteille (10).

6. Bouteille selon l'une des revendications précédentes, caractérisée en ce que :

- l'élément (40) en forme de pot a au moins dans son fond (48) une plaque ferromagnétique (42).

7.  Bouteille selon l'une des revendications précédentes, caractérisée en ce que :
    - le diamètre extérieur de l'élément (18, 50) en forme de pot correspond au diamètre extérieur de la zone périphérique s'étendant plus en haut de la bouteille.

8.  Bouteille selon l'une des revendications 1 à 7, caractérisée en ce que :
    - la nervure (16) a sur sa surface supérieure (26) une surface d'encoche s'étendant essentiellement radialement, qui vient en prise avec une surface d'encoche (32) existant radialement sur la saillie (30).

9.  Bouteille selon l'une des revendications précédentes, caractérisée en ce que :
    - des coins libres et arêtes de la bouteille en particulier dans son espace interne et de l'élément en forme de pot ou d'anneau sont arrondis ou tombent en oblique vers l'intérieur.

10. Bouteille selon l'une des revendications précédentes, caractérisée en ce que :
    - au moins une saillie (30) de type moulure est réalisée sur la face interne de la paroi du pot (24), pour s'enclencher avec la nervure inférieure (16).

11. Bouteille selon l'une des revendications précédentes, caractérisée en ce que :
    - sur la face interne de la paroi de pot (24) sont mis en place quatre saillies (30) de type moulure.

Fig. 1

10

12

14

III

18

Fig. 2

10

1

16

14

IV

Fig. 3

17

22

34

24

14

30

16

26

32

16

28

18

Fig. 4

20

14

17

26

28

16

Fig. 5

Fig. 6

Fig. 7

Fig. 8

Fig. 9

Fig. 10

Fig. 11

Fig. 12